## Europäisches Patentamt
## European Patent Office
## Office européen des brevets

(19)

(11) Publication number: **0 086 883**

**A1**

(12)

# EUROPEAN PATENT APPLICATION

(21) Application number: **82111141.6**

(51) Int. Cl.³: **A 61 B 1/00**

(22) Date of filing: **02.12.82**

(30) Priority: **11.12.81 ES 507900**

(43) Date of publication of application:
**31.08.83 Bulletin 83/35**

(84) Designated Contracting States:
**AT BE CH DE FR GB IT LI LU NL SE**

(71) Applicant: **Pichel Moure, Carlos**
**Nicaragua, 2**
**La Coruna(ES)**

(72) Inventor: **Pichel Moure, Carlos**
**Nicaragua, 2**
**La Coruna(ES)**

(74) Representative: **Prato, Roberto et al,**
**c/o Ingg. Carlo e Mario Torta Via Viotti 9**
**I-10121 Torino(IT)**

(54) Osteoscope for carrying out the nailing of the fractures.

(57) An osteoscope (1) is proposed for realizing the nailing of fractures "under closed sky" through endoscopic control. The apparatus is equipped with a head (2) crossed by various channels (for optical fibres for illumination purposes, for drainage, for sucking, for instruments introduction and for that of guiding pegs) and equipped with lenses, articulations and a case (7) with mechanisms. The equipment further contains a guiding wheel (6), a visualizer (8) in the case and protruding from it. The apparatus can be connected for uncontamination purposes with a closed circuit television, enabling the entire surgical team to observe the operation without touching the apparatus. Finally the apparatus contains a sheath (11) of semi-rigid type inside which the apparatus can be slipped.

FIG. 1

EP 0 086 883 A1

- 1 -

OSTEOSCOPE FOR CARRYING OUT THE NAILING OF THE FRACTURES

The present invention refers to an osteoscope for carrying out the nailing of the fractures which are not in the open, through endoscopic control.

Inspite of recent progress in the therapeutics of fractures - electrical stimulation of the bones consolidation, use of functional plasters based on a hydrostatic principle or on a uniform compactness, biocompression principles, control of consolidation through detectors, investigation with bio-materials - and notwithstanding the increasing distrust towards the efficiency of the metallic osteo-synthesis (compression plates) the intramedullary nailing of Küntacher remains an indiscussed method for treating of diaphysial fractures, especially of those having a cross direction or a short skew one, localized at the level of the third portion of the femur and, in lesser cases, of the tibia and of the humerus.

The intramedullary fastening with pegs with flexible stems ( sheaf of Hackental, nails of Ender) get day after day a larger diffusion in the treatment of humerus and femur neck

Tractures. Other fastenings with pegs (ulna, radius) are more rarely used, but remain valid in the treatment of the forearm bone fractures,in the case of children.

These forms of ostesynthesis of intramedullary type have reached their maximum prestige level now that it is possible to carry them out "under closed sky", viz. without opening the fracture focus. I.e. by introducing the nails or pegs from an inlet point placed at a certain distance slipped into,away from the fractured fragments,through a radioscopic control. The technique called "under closed sky" reduces the risks of infections because it does not expose directly the bone fragments and improves the conditions of consolidation with respect to the direct surgery intervention into the fracture focus,thus sparing the periosteum circulation.

The intramedullary nailing "under closed sky" of the diaphysial fractures was described by Gerald Küntacher in 1940,

but the notable radiation risks involving the surgeons , the ancillary personnel and to a lesser extent the patients themselves (let us remind that radiations are accumulative) limited the diffusion of the method, till the advances in electronics allowed the putting on the market of the image intensifiers which allow the radioscopic vision with the help of lower radiations with respect to the conventional X ray tubes.

The radiations received by surgical personnel may however border on dangerous limits considering the frequency in the use

of intensifiers (due to the lower risks involved and to the increase in the number of operations "under closed sky".
 00286883

In  principle severe measures of radiological protection should be sufficient for avoiding the radiation risks but the heavy lead aprons and the difficulties connected with a systematic dosimetry make it difficult to respect strictly the radio-protective regulations.

In order to expose the action on a human body we report textually some sections dealing with long term effects of radiation of the Treaty of Internal Medicine of Harrison:
"       The radiation alters the "information system" of the somatic and germinal prolife ant cells . In this way the tissues which perpetuate the blood, the gastroenteric apparatus, the skin, the crystalline, the gonads and other structures transmit to the progeny "a bad and inadecuate information" which entails, after a long time, a somatic illness, e.g. cancer, cataracts, degenerations or an unspecified shortening of life. It was possible to measure the degree of leucemia produced through radiations in groups of human beings; it is supposed, although not proven, that there is no threshold and that production of leucemia increases with the dose. However the increased exposure of public (to the danger) derives from the medical application od different types of radiation (especially for X ray diagnosis). If the previous assertion is correct , this use of radiations should be responsible for the increase of the chances of leucemia and other diseases. This implies for the MD an extreme caution in effecting radiological examinations on his patients, unless there exists a clear indication for carrying out a diagnosis.

There is no doubt that radiation can produce mutations in genes, which are structures forming the centres of information and transmission for heredity. Not all the mutations are prejudicial, but there are enormous probabilities that the change might be detrimental to the species. Not all the mutations imply immediate effects. The problem consists not only in the probability of an increase in the number of deformed creatures or of creatures evidencing lesions, but also in the probability of changes entailing undesired characteristics, like the shortening of life, the decrease in fertility, the general increase of physical and mental diseases and also the augmentation of abortions and dead-born children."

Besides the strictly biological effects, the use of radiations also involves psychological factors, and also factors connected with working activities and economical ones, which are all to be added to the previous ones,when considering the employing of radioscopic operations as a necessary evil in the field of traumatology and orthopedy..

It would be without any doubt a progress to avail ourselves of the method of osteosynthesis in intramedullary field,

thus avoiding risks and drawbacks mentioned hereinbefore.

To this basic scope we conceived an osteoscope through which it is possible to carry out the procedure of nailing the fractures "under closed sky" through an endoscopic control, which

0086883

is expounded hereinafter:

Endoscopy as technique for observing different organs of the human body represents today a routine technique in most of world hospitals and has reached a notable degree of technological development with various applications, i.e. bronchoscopy, gastroscopy, laparatoscopy, just as some instances. The first attempts to visualize osteo-articular/structures were made by Takagi (1913) and Bircher (1921). As a matter of fact arthroscopy began to be used in clinical field as from 1970, having then at its disposal the arthroscope of Estenabe, who published with Takeda and Ikeuchi the first "Atlas of Arthroscopy" in 1957.

In the last years and according to new optical techniques (incorporation of cold light sources, luminous fibres etc.) and to miniaturization, the method further advanced and the field of utilization was broadened and reached practically the stage of endoscopic surgery . Today one performs operations (with direct vision) using special instruments introduced along the endoscopes.

Until now and in the domain of traumatology and orthopedy endoscopy was only used for carrying out observations and operating procedures on articulations. The basis of the nailing procedure consists in the utilization of the principles of endoscopy in order to visualize directly the medullary cavity of long bones.

From a theoretical viewpoint, the difficulties of the realization of osteoscopy of the intra-medullary type consist in the following:

- The diaphysial cavity is occupied by bony medulla.

- The medullary cavity has no natural inlet orifice.

- The bones are rigid structures with curves.

These difficulties can be overcome through the design of the osteoscope and the procedure of utilization which is to be recorded.

The subject of the invention consists therefore in an osteoscope for the realization of the nailing of bony fractures formed by a semi-rigid structure allowing the fitting to the shape of the bone.

Inside said semi-rigid shell the following basic components are housed:

- A channel for optical fibres for lighting scopes.

- A channel for optical fibres for visual scopes

- A channel for drainage.

- A channel for introducing instruments and guiding pegs.

To meet the miniaturization requirements and a peculiar use of the osteoscope, some of these channels can be eliminated. For instance a single channel can be used in subsequent stages for draining, sucking and instruments introduction.

Dimensions and shape of the osteoscope itself and the relevant channels thereof can vary according to the bone to which it will be applied.

The end portion of the osteoscope is equipped with a head element to which the different channels are connected and which comprizes a lens with different characteristics

0086883

connected in turn with the channel for visual fibres, and lenses for the illuminating channel.

Between the arm of the osteoscope or a portion thereof which is being introduced into the bone and the head portion there is an articulated system which allows its mobility.

As far as the main axis of the osteoscope, the same is formed by the channels used for the introduction of instruments and guiding pegs. It ends with a steering wheel which makes easier the handling.

As far as the optical, illuminating, draining and sucking systems are concerned, they leave laterally the axle of the apparatus and end in a box containing mechanisms from which starts the visual device containing  connections for sources of cold light, vacuum, and fluid under pressure.

The visualising system can be foreseen for its connection with a closed circuit television, permitting the simultaneous observation by the whole surgical team, avoiding in this way the risks of contamination of the apparatus due to the direct contact with the eye.

Finally it must be said that the ancillary instrumentation of the osteoscope is composed by a stiff sheath through which the apparatus can be slipped    , thus permitting the manipulation of the bony fragments for specific applications, and a guiding peg with moving head, which can be angled with respect to its longitudinal axis for some specific applications.

These two basic elements of instrumentation (stiff sheath

**0086883**

and guiding peg with moving head) can be integrated by an endo-

scopic spoon for the cleaning of the fracture focus and by a   rod or an endoscopic brush.

In order to better understand the range of the invention, we enclose a set of drawings with some figures describing visually the osteoscope and its application . On such drawings we have:

On figure 1a aschematic view of the osteoscope, built in accordance with the invention.

On figure 2a the use of the osteoscope in a process of intra-medullary nailing "under closed sky" with endoscopic control.

On the mentioned figures the digital references

corespond to the following parts and elements:

1. - Osteoscope

2. - Head piece

3. - Arm of the osteoscope (1)

4. - Collar

5. - Main axis of the osteoscope (1)

6. - Steering wheel

7. - Case with mechanisms

8. - Visor or visual device

9. - Connections

10. - Guide

11. - Stiff sheath

12. - Moving head of the guide (10)

13. - Main trocanter

14. - Visual field

15. - Visual fibres

16. - Fragments of broken bone

17. - Fragments of broken bone

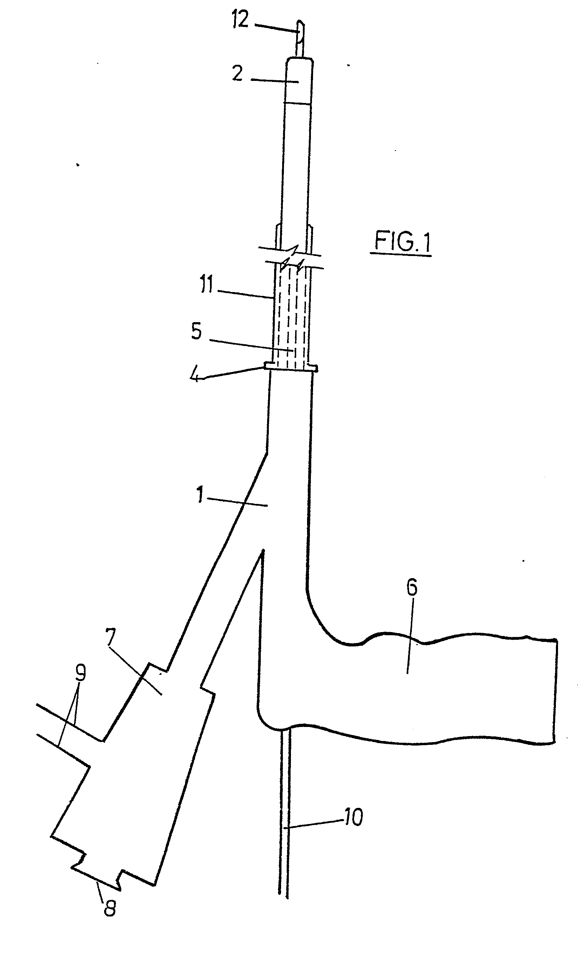

By looking at the above mentioned figures and with practical reference to figure 1a, one can observe the osteoscope (1) itself, formed by a semi-rigid shell crossed by channels (one for optical fibres, serving for illumination; one for visual optical fibres; one for drainage; one for sucking and one for the introduction of instruments and guiding pegs).

The front end portion of the osteoscope (1) is composed by a head (2) equipped in its front part by lenses in connection with the channels containing visual fibres and illuminating ones as well as orifices into which disembogue remaining channels, whilst its back portion is connected with the arm (3) or branch located between the head (2) and the collar (4), it being planned that such connection can be realized through a system of articulations (not appearing on the drawing) permitting to the head piece (2) to assume an angular position in regard to the main axis of the apparatus (5), materialized by the channel used for the introduction of instruments and guiding pegs. The osteoscope is finally completed by a steeting wheel (6) which makes easier its manipulation or handling, whilst in a divergent position with respect to the axis or axle and protruding upwards in backward direction, there is a case with mechanisms (7) from which the visor (8) is projecting , said case containing

0086883

*the connections (9) for the sources of cold light, of vacuum and*
of fluid under pressure.

The osteoscope (1) is completed  with ancillary instruments
comprizing. a stiff sheath (11) through which the instrument can
be slipped    , allowing its handling, a guiding peg. (10)
which ends frontally into the moving head (12) which is
.articulated in order to allow the angulation of the moving head (12).

In order to illustrate better the use of the above described
osteoscope  we select as example  the nailing of a femur fracture,
so that slight modifications may permit its utilization for other
long bones, such modifications occurring in technical and
instrumental fields.

To better clear up the matter and in order to prove the
advantages offered by the osteoscope, we shall describe before
all the conventional procedure for nailing "under closed sky"
under radioscopic control.

The original description by C.Küntscher   of the technique
of nailing "under closed sky"  includes many further sections
but can be summarized essentially as follows:

1) Placing the patient inside an adequate equipment
in order to effect the traction of the fracture  and to position
correctly the image amplifier,thus obtaining radioscopic projections
of orthogonal type,made in sequence, of the fracture focus.

2) Incision on the main trocanter. Perforation and
magnification of an orifice on the upper rim thereof, in order
*to be able to reach the medullary cavity.

3) Introduction through the orifice of a wire guiding into the medullary cavity.

4) Radioscopic control of the correct introduction of the guide into the fragment located nearby.

5) Reduction of the fragments of the fracture through external steps.

6) Introduction of the guide into the distal fragment of the fracture.

7) Radioscopic control of the proper positioning of the guide through two projections.

8) Magnification of the medullary cavity through the use of flexible grooved miller, evidencing an increasing diameter, lead along the guiding peg.

9) Introduction of the previously selected intra-medullary nail.

10) Extraction of the guiding wire.

The point of paramount importance of the above quoted procedure is the introduction in a correct way of the wire, which threads both fragments  and guides the other instruments of the equipment.

We shall now describe the nailing "under closed sky" under endoscopic control , using the osteoscope forming the object of the present invention :

1) Preventive ischemia  for stopping patient's bleeding. Putting the patient in an adecuate position.

2) Incision on main trocanter (13). Perforation with

0086883

a punch of an orifice on the upper rim thereof.

3) Amplifying same through the use of a rasp in the direction of the medullary channel, digging a channel in the spongy trocanter tissue. The outflow through the orifice of blood and drops of grease shows that the medullary cavity has been reached.

4) Cleaning of the medullary cavity through the use of a spoon with long handle or of the medullary brush.

5) Introduction of a probe or sound till the fracture focus. Sucking of the fracture hematoma.

6) Introduction of the stiff sheath (11) of the osteoscope (1) into the proximal fragment. The portion of such a sheath protruding from the operation wound , permits to move the proximal fragment.

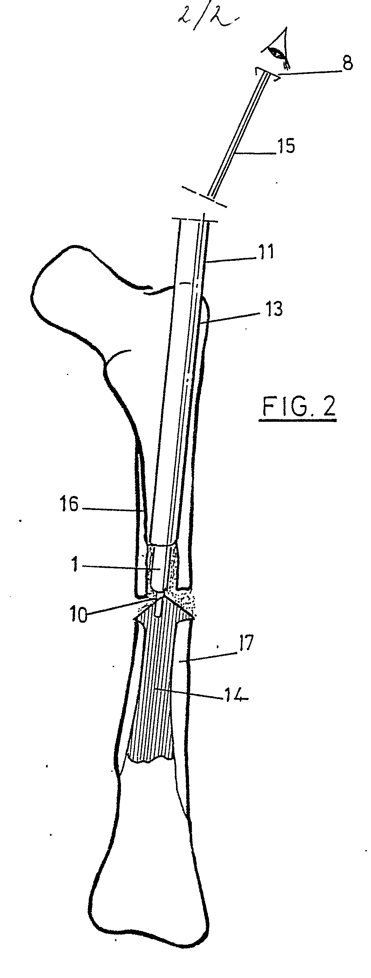

7) Progressive introduction of the osteoscope (1) through the sheath (11), till reaching, under visual control, the fracture focus (please observe on figure 2a the visual field (14) corresponding to the shadowed zone, as well as the visual fibres (15) ).

8) By handling the sheath (11) the medullary orifice of the distal fragment is looked for. Washing and sucking the residuals of the hematoma in the fracture focus. The introduction of the endoscopic spoon shall allow more efficiently the cleaning of the coagulated blood or of detrital elements of the tissue which were not moved by pressure washing.

9) Introduction of the guiding peg (10) under direct

0086883

visual control through the osteoscope. The possibility of putting the head (12) of the  guide (10) in angular positions facilitates this. The vision through a closed circuit television permits to an assistant to collaborate in the process through an external manipulation in the lining up  of the fragments, bearing the numbers (16 & 17).

10) Introduction of the osteoscope (1) into the distal

fragment by slipping along the guiding peg. Washing and sucking the intramedullary grease or use of the endoscopic brush if necessary.  Going forward of the osteoscope  till tubercles of the spongy bone of the distal epiphysis of the femur becomes visible. The length of the introduced osteoscope determines the size of the nail.

11) Extraction of the guiding peg and replacement of same by a conventional guide . Progressive extraction of the osteoscope till it reaches the level of the fracture focus.

Endoscopic control of the correct reduction of the fracture.

12) Extraction of the osteoscope.

After this the process continues in a conventional way.

According to what expounded , the advantages of the osteoscope can be summarized as follows:

1. - Radioscopic examination appears no longer necessary so that radiation risks are diminished .

This concerns:

a) The surgeons and the personnel of the operating team.

b) The patient himself or herself. Under normal con- ditions radiation absorbed by the patient is not dangerous for his or her health, but can damage the embryo in case of pregnant women.

2. - Permits a tridimensional vision of the fracture focus, whilst the amplifier permits only flat images. This will facilitate the fracture reduction.

3. - The sucking of the bony medulla and the emptying of the diaphysis cavity  probably diminishes the intra-medullary pressure and the risk of fat embolism.


4. - The positioning of the patient in the conventional procedure is difficult to achieve and complex, due to the need to be in conditions to turn the tube of the amplifier of images without obstacles or interpositions, in order to obtain orthogonal projections. These drawbacks are avoided by using our procedure and the relevant improvements connected with it.

5. - Although the technique was conceived initially for the treatment of diaphysis fractures, it can be applied to other cases like observation of tumours and pathological processes of intra-medullary kind or other forms of intra-diaphysial surgery.

CLAIMS                                    0086883 ⁺

1.-    Osteoscope for realizing the nailing of fractures "under
closed sky", through endoscopic control, essentially characterized
by the fact that it is formed by a semi-rigid shell crossed inside
it by a channel for optical fibres for illumination purposes, a
channel for drainage, a channel for sucking and a channel for
the introduction of instruments and guiding pegs, its peculiar
aspect being that the extreme front end of said shell is equipped
with a head connected with the a/m channels and includes a lens
connected with visual fibres and lenses for the illumination
channel, so that between the extreme back portion of said shell
of semi-rigid kind and said head appears a system of articulations
which allow the mobility of the apparatus, whilst behind such
semi-rigid shell the body of the apparatus is prolonged and
is building an extension forming on one side a lower guiding
wheel for handling purposes and on the other side a projection
protruding upwards and aslant, realizing in this way a case
containing mechanisms from which a visor or visualizer protrudes
and which case contains further connections for sources of cold
light, vacuum and fluid under pressure, the apparatus finally
having a central axle or axis with a channel for the introduction
of instruments and guiding pegs.

2.-    Osteoscope for realizing the nailing of fractures "under
closed sky", through endoscopic control, according to the claim
1, characterized by the fact that in view of the needs for
miniaturization and for peculiar uses of the apparatus, some channels may be suppressed and be amalgamated into a single one ,
to be used successively for different operations, like drainage,
sucking and instruments introduction.

3.-     Osteoscope for realizing the nailing of fractures "under
closed sky", through endoscopic control, according to the claim
1, characterized by the fact that the system for vision can be
possibly connected with a closed circuit television, thus permitting the observation at the same time by the entire surgical
team, whilst avoiding the dangers of contamination of the apparatus
itself.

4.-     Osteoscope for visualizing  the nailing of fractures "under
closed sky" , through endoscopic control, according to claim 1,
characterized by the fact that ancillary instrumentation is
formed by a stiff sheath through which the semi-rigid shell
of the apparatus can be slipped and a guiding·peg with moving head,
which can be put in angular positions with respect to the
longitudinal axis.

12

2

FIG.1

11

5

4

1

7

9

6

8

10

0086883

FIG. 2

## DOCUMENTS CONSIDERED TO BE RELEVANT

EP 82111141.6

| Category | Citation of document with indication, where appropriate, of relevant passages | Relevant to claim | CLASSIFICATION OF THE APPLICATION (Int. Cl. 3) |
|---|---|---|---|
| Y | US - A - 4 146 019 (M. BASS, R.M. DWYER)<br><br>* Totality *<br><br>-- | 1.2 | A 61 B 1/00 |
| Y | US - A - 3 091 235 (O.W. RICHARDS)<br><br>* Fig. 1,3,5; column 6, line 18 - column 8, line 69 *<br><br>-- | 1,2 | |
| A | GB - A - 719 538 (A.P. GUTTMAN)<br><br>* Totality *<br><br>-- | 1,4 | |
| A | US - A - 4 277 168 (T. OKV)<br><br>* Fig. 1; column 2, lines 27-40 *<br><br>-- | 1,3,4 | |
| A | DE - A1 - 3 004 335 (MEDOS KENKYUSHO)<br><br>* Fig.; page 5, last paragraph - page 7, first paragraph *<br><br>-- | 1,2 | TECHNICAL FIELDS SEARCHED (Int. Cl. 3)<br><br>A 61 B 1/00 |
| A | EP - A2 - 0 029 281 OLYMPUS OPT.)<br><br>* Fig. 1,2a,2b; page 2, line 19 - page 3, line 17 *<br><br>-- | 1,2 | |
| A | GB - A - 2 068 139 (R.W. CARSON)<br><br>* Totality *<br><br>-- | 1 | |

The present search report has been drawn up for all claims

| Place of search | Date of completion of the search | Examiner |
|---|---|---|
| VIENNA | 08-06-1983 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

## DOCUMENTS CONSIDERED TO BE RELEVANT

| Category | Citation of document with indication, where appropriate, of relevant passages | Relevant to claim |
|---|---|---|
| A | <u>US - A - 899 175</u> (W. MEYER)<br>  * Totality; especially page 2, lines 44-55 * | 1,4 |
| A | <u>US - A - 3 871 358</u> (N. FUKUDA)<br>  * Fig. 1,3; column 2, lines 4-14 * | 4 |

CLASSIFICATION OF THE APPLICATION (Int. Cl. ³)

TECHNICAL FIELDS SEARCHED (Int. Cl. ³)